# EUROPEAN PATENT SPECIFICATION

(11) **EP 2 427 214 B1**
(45) Date of publication and mention of the grant of the patent: **13.03.2013**
(21) Application number: 09790883.4
(22) Date of filing: 28.07.2009
(51) Int. Cl.: A61K 47/36, A61K 47/48, A61K 47/40

(54) **TOPICAL SOLUTION FORMULATIONS CONTAINING A CORTICOSTEROID AND A CYCLODEXTRIN**
TOPISCHE LÖSUNGSFORMULIERUNGEN MIT EINEM KORTIKOSTEROID UND EINEM CYCLODEXTRIN
FORMULATIONS DE SOLUTION TOPIQUE CONTENANT UN CORTICOSTÉROÏDE ET UNE CYCLODEXTRINE

(30) Priority: 08.05.2009 US 437895
(43) Date of publication of application: 14.03.2012
(73) Proprietor: Novartis AG, 4056 Basel (CH)
(72) Inventor: CASTILLO, Ernesto J., Arlington Texas 76016 (US); ZHANG, Huixiang, Fort Worth Texas 76137 (US); STAFFORD, Glenn D., Jr., White Settlement Texas 76108 (US); HAN, Wesley Wehsin, Arlington Texas 76006 (US)
(74) Representative: Teipel, Stephan
(86) International application number: PCT/US2009/051955
(87) International publication number: WO 2010/128983

(56) References cited:
- US-A1- 2003 232 089
- US-A1- 2007 020 299

## Description

### BACKGROUND OF THE INVENTION

This invention relates to topically administrable solution formulations containing a corticosteroid and a cyclodextrin.

Many corticosteroids are known, one of which is dexamethasone. Both solution and suspension compositions containing dexamethasone as the sole active agent are marketed. The solution compositions contain dexamethasone in the form of dexamethasone sodium phosphate. The suspension formulations contain dexamethasone in the form of dexamethasone alcohol. See Ophthalmic Drug Facts '99, Facts and Comparisons, St. Louis, Mo. (1999), p. 87. Additionally, aqueous anti-inflammatory/anti-infective combination products containing dexamethasone are currently marketed. See Ophthalmic Drug Facts '99, Facts and Comparisons, St. Louis, Mo. (1999), p. 121 - 122. The only such combination product identified as a solution is a neomycin sulfate/dexamethasone sodium phosphate solution product.

Solution compositions containing water-insoluble forms of dexamethasone (i.e., forms other than dexamethasone phosphate) must contain a solubilizing agent. Cyclodextrins are one type of solubilizing aid that has been used with steroids. See, for example, U.S. Patent No. 4,383,992; U.S. Patent No. 5,229,370; and European Patent No. 0 326 196 B1.

US 2007/020299 A1 discloses an inhalable nasal composition comprising a corticosteroid, a β-cyclodextrin, a second active ingredient, an emulsifying agent such as xanthan gum and other excipients.

To be commercially viable, solution compositions must remain physically stable over extended periods of time to permit manufacture, handling, storage, shipping and a reasonable shelf-life.

### SUMMARY OF THE INVENTION

The present invention provides solution compositions of water-insoluble corticosteroids. The present compositions contain a cyclodextrin as a solubilizing agent. In addition, the compositions contain xanthan gum in an amount sufficient to enhance the physical stability of the compositions.

Among other factors, the present is based on the finding that solution compositions containing a corticosteroid, a cyclodextrin and xanthan gum possess superior physical stability compared to similar formulations that lack xanthan gum or that contain polyethylene glycol instead of xanthan gum.

### DETAILED DESCRIPTION OF THE INVENTION

Unless indicated otherwise, all ingredient amounts presented as a percentage are in weight/weight units, % (w/w).

The corticosteroid ingredient of the present invention may be any pharmaceutically acceptable corticosteroid that is not sufficiently soluble in water to provide a target corticosteroid concentration in a solution composition. Suitable corticosteroids include, but are not limited to, dexamethasone, fluorometholone, prednisolone, loteprednol and rimexolone. A preferred corticosteroid is dexamethasone in the form of dexamethasone alcohol or dexamethasone acetate. The corticosteroid ingredient will comprise 0.01 - 0.3%, preferably 0.05 - 0.2%, and most preferably about 0.1%.

The cyclodextrin ingredient in the compositions of the present invention may be any pharmaceutically acceptable cyclodextrin. Many cyclodextrins are known, including, but not limited to those classified as β-cyclodextrin derivatives, γ-cyclodextrin derivatives and sulfated cyclodextrin derivatives. A preferred cyclodextrin is hydroxypropyl-β-cyclodextrin. The amount of cyclodextrin ingredient included in the compositions of the present invention will depend on the concentration of corticosteroid. The amount of cyclodextrin should be enough to solubilize all of the selected corticosteroid so that the composition is administered to a patient as a solution. Generally, the amount of cyclodextrin contained in the compositions of the present invention will be 1 to 15%, preferably 2 to 10%, and most prefereably 4 to 7%.

In addition to the corticosteroid and cyclodextrin, the compositions of the present invention contain xanthan gum. Xanthan gum is a well-known polysaccharide that is commercially available from a variety of sources. The amount of xanthan gum contained in the compositions of the present invention will depend upon the amounts of the corticosteroid and cyclodextrin ingredients in the composition, but ranges from 0.1 to 0.6%, preferably 0.1 - 0.4%, and most preferably, 0.2 - 0.3%. The compositions contain an amount of xanthan gum sufficient to enhance the physical stability of the composition relative to a similar composition lacking xanthan gum.

The compositions of the present invention have a pH from 4 - 8. pH can be adjusted with NaOH/HCl or other pH adjusting agents known in the art. The compositions of the present invention may contain one or more buffering agents.

The solution compositions of the present invention optionally comprise a second active ingredient. Any pharmaceutically active compound that is suitable for ophthalmic, otic or nasal administration may be used. Such active ingredients include, but are not limited to fluoroquinolone antibiotics, such as ciprofloxacin, moxifloxacin, and gatifloxacin. The fluoroquinolone can be present in any pharmaceutically acceptable form such that it is in solution in the composition that is administered to a patient. A preferred fluoroquinolone antibiotic is ciprofloxacin. A preferred form of ciprofloxacin is ciprofloxacin hydrochloride, monohydrate. If present, the fluoroquinolone ingredient will comprise 0.1 - 1% of the compositions of the present invention. In the case where the fluoroquinolone is ciprofloxacin, the preferred amount of ciprofloxacin in the compositions of the present invention is 0.3%. In the case where the fluoroquinolone is moxifloxacin, the preferred amount of moxifloxacin in the compositions of the present invention is 0.5%.

In addition to the active agent(s), the cyclodextrin and the xanthan gum ingredients, the compositions of the present invention may contain one or more conventional excipients, including, but not limited to, tonicity agents, preservatives, antioxidants, chelating agents and preservative enhancing agents.

The compositions may contain an ionic or nonionic tonicity agent. The amount of tonicity agent will depend on the desired tonicity for the final formulation, but will generally be an amount sufficient to cause the formulations to have an osmolality of 100 - 600 mOsm. In cases where the composition is intended for topical ophthalmic use, the composition preferably has an osmolality of 250 - 350 mOsm.

The compositions of the present invention may be prepared without a preservative as a "unit-dose" or "unpreserved" formulation. If a preserved or "multi-dose" formulation is desired, the formulations may contain an ophthalmically, otically or nasally acceptable preservative, such as benzyl alcohol or quaternary ammonium halides. Quaternary ammonium halide preservatives are preferred. Suitable quaternary ammonium halide preservatives include polyquaternium-1 and benzalkonium halides. Preferred benzalkonium halides are benzalkonium chloride ("BAC") and benzalkonium bromide. In general, the amount of the preservative ingredient will range from 0.005 - 0.2. In the case where the preservative is BAC, it is preferably present at a concentration of 0.01%. In the case where the preservative is polyquaternium-1, it is preferably present at a concentration of 0.005%.

If desired, a chelating agent may also be added to the formulations of the present invention. Suitable chelating agents include edetate disodium ("EDTA"); edetate trisodium; edetate tetrasodium; and diethyleneamine pentaacetate. Most preferred is EDTA. The chelating agent, if any, will typically be present in an amount from 0.001 - 0.2%. In the case of EDTA, the chelating agent is preferably present at a concentration of 0.01%.

In the case of preserved or multi-dose formulations, the solution formulations of the present invention may contain boric acid, as a component of a buffer and/or as a preservative adjunct, typically in an amount from 0.1 - 1.5%.

The solution formulations of the present invention are intended for topical administration to the eye, ear or nose.

The following examples are intended to illustrate, but not limit, the present invention.

### Example 1

The formulations shown in Table 1 were prepared as follows.
1. In a suitable container dissolve in the following order the respective amount of hydroxy-propyl-β-cyclodextrin, dexamethasone, ciprofloxacin and EDTA in purified water (approx. 50% of batch weight).
2. Add the respective amount of any xanthan gum stock solution (1.2%) or polyethylene glycol until total dispersion.
3. Add the required amounts of sodium chloride and benzalkonium chloride.
4. Increase the batch weight to 90% of final batch weight.
5. Adjust pH with HCl and/or tromethamine solutions to 4.5 +/- 0.2
6. QS to final batch weight with purified water.
7. Autoclave formulation for 30 min (standard liquid cycle) or filter through a 0.22 µm filter.

**TABLE 1**

| | A | B | C | D | E | F |
|---|---|---|---|---|---|---|
| Ciprofloxacin HCl-H₂O | 0.35% | 0.35% | 0.35% | 0.35% | 0.35% | 0.35% |
| Dexamethasone | 0.1% | 0.1 % | 0.1 % | 0.1 % | 0.1 % | 0.1 % |
| HPBCD | 5% | 5% | 5% | 5% | 5% | 5% |
| Edetate Disodium | 0.01% | 0.01% | 0.01 % | 0.01 % | 0.01% | 0.01% |
| NaCl | --- | 0.6% | 0.6% | 0.6% | 0.6% | 0.6% |
| Polyethylene glycol 400 | --- | --- | 0.25% | --- | --- | --- |
| Polyethylene glycol 3350 | --- | --- | --- | --- | 0.25% | --- |
| Polyethylene glycol 8000 | --- | --- | --- | --- | --- | 0.25% |
| Xanthan qum | --- | --- | --- | 0.25% | --- | --- |
| Benzalkonium Chloride | 0.01% | 0.01% | 0.01% | 0.01% | 0.01% | 0.01% |
| Tromethamine/HCl | q.s. pH 4.5 ± 0.2 | q.s. pH 4.5 ± 0.2 | q.s. pH 4.5 ± 0.2 | q.s. pH 4.5 ± 0.2 | q.s. pH 4.5 ± 0.2 | q.s. pH 4.5 ± 0.2 |
| Purified Water | q.s. 100 | q.s. 100 | q.s. 100 | q.s. 100 | q.s. 100 | q.s. 100 |
| | | | | | | |
| Sterilization | Filter | Filter | Filter | Autoclave | Filter | Filter |

### Example 2

Formulations A - F were evaluated to determine whether they were physically stable. Samples of each formulation (5 mL fill in clear glass scintillation vials, duplicates) were placed in a refrigerator (0 °C), pulled at the indicated time points and their physical appearance noted. The results are shown in Table 2.

**TABLE 2**

| Formulation | 1 day | 7 days | 14 days | 28 days |
|---|---|---|---|---|
| A | Clear, no precipitate | Clear, some crystals | Clear, precipitate | Clear, heavy precipitate |
| B | Clear, no precipitate | Clear, some crystals | Hazy, precipitate | Hazy, lots of crystals |
| C | Clear, no precipitate | Clear, no precipitate | Clear, some crystals | Hazy, crystals suspended |
| D* | Hazy, no precipitate | Hazy, no precipitate | Hazy, no precipitate | Hazy, no precipitate |
| E | Clear, no precipitate | Clear, no precipitate | Clear, few crystals | Clear, some crystals |
| F | Clear, no precipitate | Clear, some crystals | Hazy, precipitate | Hazy, lots of crystals |

| | | | | |
|---|---|---|---|---|
| * Initial formulation is hazy | | | | |

The results in Table 2 show that in each of Formulations A - C, E and F precipitates or crystals were observed by 14 days, and in some cases after just 7 days. In contrast, no precipitates or crystals were observed in Formulation D after 28 days.

### Example 3

A representative composition according to the invention is shown below.

| **Ingredients** | **w/w%** |
|---|---|
| Moxifloxacin HCl | 0.545 |
| Dexamethasone Alcohol | 0.1 |
| HPβCD | 5.0 |
| Xanthan Gum | 0.25 |
| NaCl | 0.3 |
| Boric Acid | 0.64 |
| Hydrochloric Acid and/or Sodium Hydroxide | q.s. pH to 5.5 ± 0.2 |
| Purified Water | q.s. to 100 |

The embodiments described above are therefore considered to be illustrative in all respects and not restrictive, the scope of the invention being indicated by the appended claims rather than by the foregoing description.

## Claims

1. An ophthalmic, otic or nasal composition consisting of
a) a pharmaceutically effective amount of a water-insoluble corticosteroid,
b) a cyclodextrin in an amount sufficient to solubilize the corticosteroid,
c) xanthan gum in an amount of 0.1 to 0.6 % (w/w), and
d) water,
wherein said composition is a liquid and has an osmolality from 100 to 600 mOsm.

2. The composition of Claim 1 wherein the corticosteroid is selected from the group consisting of dexamethasone; fluorometholone; prednisolone; loteprednol; and rimexolone.

3. The composition of Claim 1 wherein the pharmaceutically effective amount of the water-insoluble corticosteroid is 0.01 - 0.3% (w/w).

4. The composition of Claim 3 wherein the pharmaceutically effective amount of the water-insoluble corticosteroid is 0.05 - 0.2% (w/w).

5. The composition of Claim 1 wherein the cyclodextrin is hydroxypropyl-β-cyclodextrin.

6. The composition of Claim 1 wherein the amount of cyclodextrin is 1 to 15% (w/w).

7. The composition of Claim 1 wherein the osmolality is 250 to 350 mOsm.

8. The composition of Claim 1 wherein the amount of xanthan gum is 0.1 - 0.4%(w/w).

9. The ophthalmic, otic or nasal composition of Claim 1 consisting of
a) a pharmaceutically effective amount of a water-insoluble corticosteroid,
b) a cyclodextrin ih an amount sufficient to solubilize the corticosteroid,
c) xanthan gum in an amount of 0.1 to 0.6 % (w/w),
d) water, and
e) one or more excipients selected from the group consisting of tonicity agents; preservatives; antioxidants; chelating agents; preservative enhancing agents; buffering agents; and pH-adjusting agents,
wherein said composition is a liquid and has an osmolality from 100 to 600 mOsm.

10. The composition of Claim 9 wherein the pharmaceutically effective amount of the water-insoluble corticosteroid is 0.01 - 0.3% (w/w).

11. The composition of Claim 9 wherein the cyclodextrin is hydroxypropyl-β-cyclodextrin.

12. The composition of Claim 9 wherein the amount of cyclodextrin is 1 to 15% (w/w).

13. The ophthalmic, otic or nasal composition of Claim 1 consisting of
a) a pharmaceutically effective amount of a water-insoluble corticosteroid,
b) a cyclodextrin in an amount sufficient to solubilize the corticosteroid,
c) xanthan gum in an amount of 0.1 to 0.6 % (w/w),
d) water,
e) one or more excipients selected from the group consisting of tonicity agents; preservatives; antioxidants; chelating agents; preservative enhancing agents; buffering agents; and pH-adjusting agents, and
f) a second active ingredient suitable for ophthalmic, otic or nasal administration,
wherein said composition is a liquid and has an osmolality from 100 to 600 mOsm.

14. The composition of Claim 13 wherein the pharmaceutically effective amount of the water-insoluble corticosteroid is 0.01 - 0.3 % (w/w).

15. The composition of Claim 13 wherein the cyclodextrin is hydroxypropyl-β-cyclodextrin.

16. The composition of Claim 13 wherein the amount of cyclodextrin is 1 to 15% (w/w).

17. The composition of Claim 13 wherein the second active ingredient is a fluoroquinolone antibiotic drug.

## Patentansprüche

1. Zusammensetzung für Auge, Ohr oder Nase, bestehend aus
a) einer pharmazeutisch wirksamen Menge eines wasserunlöslichen Corticosteroids,
b) einem Cyclodextrin in einer Menge die ausreicht, das Corticosteroid löslich zu machen,
c) Xanthan in einer Menge von 0,1 1 bis 0,6 Gew.-% und
d) Wasser,
wobei die Zusammensetzung eine Flüssigkeit ist und eine Osmolalität von 100 bis 600 mOsm aufweist.

2. Zusammensetzung nach Anspruch 1, wobei das Corticosteroid ausgewählt ist aus der Gruppe, bestehend aus Dexamethason; Fluormetholon; Prednisolon; Loteprednol und Rimexolon.

3. Zusammensetzung nach Anspruch 1, wobei die pharmazeutisch wirksame Menge des wasserunlöslichen Corticosteroids 0,01 - 0,3 Gew.-% beträgt.

4. Zusammensetzung nach Anspruch 3, wobei die pharmazeutisch wirksame Menge des wasserunlöslichen Corticosteroids 0,05 - 0,2 Gew.-% beträgt.

5. Zusammensetzung nach Anspruch 1, wobei das Cyclodextrin Hydroxypropylcyclodextrin ist.

6. Zusammensetzung nach Anspruch 1, wobei die Menge an Cyclodextrin 1 bis 15 Gew.% beträgt.

7. Zusammensetzung nach Anspruch 1, wobei die Osmolalität 250 bis 350 mOsm beträgt.

8. Zusammensetzung nach Anspruch 1, wobei die Menge an Xanthan 0,1 bis 0,4 Gew.-% beträgt.

9. Zusammensetzung für Auge, Ohr oder Nase nach Anspruch 1, bestehend aus
a) einer pharmazeutisch wirksamen Menge eines wasserunlöslichen Corticosteroids,
b) einem Cyclodextrin in einer Menge, die ausreicht, das Corticosteroid löslich zu machen,
c) Xanthan in einer Menge von 0,1 bis 0,6 Gew.-%,
d) Wasser und
e) einem oder mehreren Hilfsstoff(en), ausgewählt aus der Gruppe, bestehend aus Tonizitätsmitteln; Konservierungsmitteln, Antioxidationsmitteln; Chelatbildnern; Konservierungsverstärkem; Puffern und pH-Einstellmitteln,
wobei die Zusammensetzung eine Flüssigkeit ist und eine Osmolalität von 100 bis 600 mOsm aufweist.

10. Zusammensetzung nach Anspruch 9, wobei die pharmazeutisch wirksame Menge des wasserunlöslichen Corticosteroids 0,01 - 0,3 Gew.-% beträgt.

11. Zusammensetzung nach Anspruch 9, wobei das Cyclodextrin Hydroxypropylcyclodextrin ist.

12. Zusammensetzung nach Anspruch 9, wobei die Menge an Cyclodextrin 1 bis 15 Gew.-% beträgt.

13. Zusammensetzung für Auge, Ohr oder Nase nach Anspruch 1, bestehend aus
a) einer pharmazeutisch wirksamen Menge eines wasserunlöslichen Corticosteroids,
b) einem Cyclodextrin in einer Menge, die ausreicht, das Corticosteroid löslich zu machen,
c) Xanthan in einer Menge von 0,1 bis 0,6 Gew.-%,
d) Wasser,
e) einem oder mehreren Hilfsstoff(en), ausgewählt aus der Gruppe, bestehend aus Tonizitätsmitteln; Konservierungsmitteln, Antioxidationsmitteln; Chelatbildnern; Konservierungsverstärkern; Puffern und pH-Einstellmitteln, und
f) einem zweiten Wirkstoff, der für die Verabreichung an Auge, Ohr oder Nase geeignet ist, wobei die Zusammensetzung eine Flüssigkeit ist und eine Osmolalität von 100 bis 600 mOsm aufweist.

14. Zusammensetzung nach Anspruch 13, wobei die pharmazeutisch wirksame Menge des wasserunlöslichen Corticosteroids 0,01 - 0,3 Gew.-% beträgt.

15. Zusammensetzung nach Anspruch 13, wobei das Cyclodextrin Hydroxypropyl-β-cyclodextrin ist.

16. Zusammensetzung nach Anspruch 13, wobei die Menge an Cyclodextrin 1 bis 15 Gew.-% beträgt.

17. Zusammensetzung nach Anspruch 13, wobei der zweite Wirkstoff ein Fluorchinolon-Antibiotikum ist.

## Revendications

1. Composition ophtalmique, auriculaire ou nasale constituée des composants suivantes :
a) une quantité efficace au plan pharmaceutique d'un corticostéroïde insoluble dans l'eau,
b) une cyclodextrine en quantité suffisante pour solubiliser le corticostéroïde,
c) de la gomme xanthane en quantité de 0,1 à 0,6 % (p/p), et
d) de l'eau,
dans laquelle ladite composition est un liquide et a une osmolalité de 100 à 600 mOsm.

2. Composition selon la revendication 1, dans laquelle le corticostéroïde est choisi dans le groupe constitué de la dexaméthasone ; de la fluorométholone ; de la prednisolone ; du loteprednol ; et de la rimexolone.

3. Composition selon la revendication 1, dans laquelle la quantité efficace au plan pharmaceutique du corticostéroïde insoluble dans l'eau est de 0,01 à 0,3 % (p/p).

4. Composition selon la revendication 3, dans laquelle la quantité efficace au plan pharmaceutique du corticostéroïde insoluble dans l'eau est de 0,05 à 0,2 % (p/p).

5. Composition selon la revendication 1, dans laquelle la cyclodextrine est l'hydroxypropyl-ß-cyclodextrine.

6. Composition selon la revendication 1, dans laquelle la quantité de cyclodextrine est de 1 à 15 % en poids (p/p).

7. Composition selon la revendication 1, dans laquelle l'osmolalité est de 250 à 350 mOsm.

8. Composition selon la revendication 1, dans laquelle la quantité de gomme xanthane est de 0,1 à 0,4 % (p/p).

9. Composition ophtalmique, auriculaire ou nasale selon la revendication 1, constitué des composants suivantes :
a) une quantité efficace au plan pharmaceutique d'un corticostéroïde insoluble dans l'eau,
b) une cyclodextrine en quantité suffisante pour solubiliser le corticostéroïde,
c) de la gomme xanthane en quantité de 0,1 à 0,6 % (p/p),
d) de l'eau, et
e) un ou plusieurs excipients choisis dans le groupe constitué des agents de tonicité ; des conservateurs ; des antioxydants ; des agents chélatants ; des agents renforçateurs de conservation ; des agents tampons ; et des agents d'ajustement du pH,
dans laquelle ladite composition est un liquide et a une osmolalité de 100 à 600 mOsm.

10. Composition selon la revendication 9, dans laquelle la quantité efficace au plan pharmaceutique du corticostéroïde insoluble dans l'eau est de 0,01 à 0,3 % (p/p).

11. Composition selon la revendication 9, dans laquelle la cyclodextrine est l'hydroxypropyl-ß-cyclodextrine.

12. Composition selon la revendication 9, dans laquelle la quantité de cyclodextrine est de 1 à 15 % (p/p).

13. Composition ophtalmique, auriculaire ou nasale selon la revendication 1, constituée des composants suivantes :
a) une quantité efficace au plan pharmaceutique d'un corticostéroïde insoluble dans l'eau,
b) une cyclodextrine en quantité suffisante pour solubiliser le corticostéroïde,
c) de la gomme xanthane en quantité de 0,1 à 0,6 % (p/p),
d) de l'eau,
e) un ou plusieurs excipients choisis dans le groupe constitué des agents de tonicité ; des conservateurs ; des antioxydants ; des agents chélatants ; des agents renforçateurs de conservation ; des agents tampons ; et des agents d'ajustement du pH, et
f) un second ingrédient actif convenant à une administration ophtalmique, auriculaire ou nasale,
dans laquelle ladite composition est un liquide et a une osmolalité de 100 à 600 mOsm.

14. Composition selon la revendication 13, dans laquelle la quantité efficace au plan pharmaceutique du corticostéroïde insoluble dans l'eau est de 0,01 à 0,3 % (p/p).

15. Composition selon la revendication 13, dans laquelle la cyclodextrine est l'hydroxypropyl-ß-cyclodextrine.

16. Composition selon la revendication 13, dans laquelle la quantité de cyclodextrine est de 1 à 15 % (p/p).

17. Composition selon la revendication 13, dans laquelle le second ingrédient actif est un médicament antibiotique de type fluoroquinolone.
